**Europäisches Patentamt**

**European Patent Office**

**Office européen des brevets**

(19)

(11) **EP 1 295 597 A1**

(12) **DEMANDE DE BREVET EUROPEEN**

| | |
|---|---|
| (43) Date de publication:<br>**26.03.2003 Bulletin 2003/13** | (51) Int Cl.7: **A61K 31/337** |

(21) Numéro de dépôt: **02028931.0**

(22) Date de dépôt: **08.11.1993**

(84) Etats contractants désignés:
**AT BE CH DE DK ES FR GB GR IE IT LI LU NL PT SE**

(30) Priorité: **10.11.1992 FR 9213525**

(62) Numéro(s) de document de la (des) demande(s) initiale(s) en application de l'article 76 CBE:
**01101665.6 / 1 093 811**
**97117252.3 / 0 827 745**

(71) Demandeur: **Aventis Pharma S.A.**
**92160 Antony (FR)**

(72) Inventeur: **Bissery, Marie-Christine**
**94400 Vitry sur seine (FR)**

(74) Mandataire: **Le Pennec, Magali**
**Aventis Pharma S.A.**
**Direction des Brevets,**
**20 Avenue Raymond Aron**
**92165 Antony Cédex (FR)**

Remarques:
• Cette demande a été déposée le 23 - 12 - 2002 comme demande divisionnaire de la demande mentionnée sous le code INID 62.
• Cette demande a été déposée le 23 - 12 - 2002 comme demande divisionnaire de la demande mentionnée sous le code INID 62.

(54) **Compositions antitumorales contentant des derives du taxane**

(57) Combinaisons antitumorales constituées de taxol ou de Taxotère ou de leurs analogues associés à au moins une substance thérapeutiquement utile dans le traitement des maladies néoplastiques.

**EP 1 295 597 A1**

**Description**

La présente invention concerne les combinaisons du taxol, du Taxotère et de leurs analogues et de substances thérapeutiquement utiles dans le traitement des maladies néoplastiques.

Le taxol, le Taxotère et leurs analogues, qui présentent des propriétés antitumorales et antileucémiques remarquables, sont particulièrement utiles dans le traitement des cancers de l'ovaire, du sein ou du poumon.

La préparation du taxol, du Taxotère et de leurs dérivés font l'objet, par exemple, des brevets européens EP 0 253 738 et EP 0 253 739 et de la demande internationale PCT WO 9209589.

Généralement les doses utilisées, qui dépendent des facteurs propres au sujet à traiter, sont comprises entre 1 et 10 mg/kg par voie intra-péritonéale ou entre 1 et 3 mg/kg par voie intra-veineuse.

Dans US-A-7 696 923 est décrite une méthode devant permettre de sélectionner des combinaisons de substances anticancéreuses dont l'une est active avant ou pendant la phase S, tel que le taxol, et l'autre après la phase S sans qu'il soit donné pour autant des exemples de telles combinaisons. Dans WO 92/19765 est décrite une association de taxol et de cis-platine qui, d'après les résultats donnés, ne montre pas d'effet synergique.

Il a maintenant été trouvé, et c'est ce qui fait l'objet de la présente invention, que l'efficacité du taxol, du Taxotère et de leurs analogues peut être considérablement améliorée lorsqu'ils sont administrés en association avec au moins une substance thérapeutiquement utile dans les traitements anticancéreux ayant un mécanisme d'action identique ou différent de celui des dérivés du taxane.

Parmi les substances qui peuvent être utilisées en association ou en combinaison avec le taxol, le Taxotère ou leurs analogues peuvent être cités des agents alkylants tels que le cyclophosphamide, l'ifosfamide, le melphalan, l'hexaméthylmélamine, le thiotépa ou la dacarbazine, des antimétabolites tels que les analogues de pyrimidine comme le 5-fluorouracil et la cytarabine ou ses analogues tels que la 2-fluoro désoxycytidine ou les analogues d'acide folique tels que le méthotrexate, l'idatrexate ou le trimétrexate, des enzymes tels que la L-asparaginase, des modificateurs de la réponse biologique ou des inhibiteurs de facteurs de croissance tels que les interférons ou les interleukines.

Par ailleurs, l'activité des produits dépendant des doses utilisées, il est possible d'utiliser des doses plus élevées et d'augmenter l'activité en diminuant les phénomènes de toxicité ou en retardant leur apparition en associant au taxol, au Taxotère, à leurs analogues ou à leurs combinaisons avec d'autres substances thérapeutiquement actives des facteurs de croissance de type hématopoïétique tels que le G-CSF ou le GM-CSF ou certaines interleukines.

Les combinaisons ou les associations selon l'invention permettent d'éviter ou de retarder les phénomènes de résistance pléïotropique ou de "multi-drug resistance".

Plus particulièrement, l'invention concerne les associations du taxol, du Taxotère et de leurs analogues avec le cyclophosphamide, le 5-fluorouracil, la doxorubicine.

L'efficacité améliorée d'une combinaison selon l'invention peut être mise en évidence par la détermination du synergisme thérapeutique.

L'efficacité d'une combinaison selon l'invention peut aussi être caractérisée par l'addition des actions de chaque constituant.

Une combinaison manifeste un synergisme thérapeutique s'il est thérapeutiquement supérieur à l'un ou l'autre des constituants utilisé à sa dose optimale [T.H. CORBETT et coll., Cancer Treatment Reports, 66, 1187 (1982)].

Pour mettre en évidence l'efficacité d'une combinaison, il peut être nécessaire de comparer la dose maximale tolérée de la combinaison à la dose maximale tolérée de chacun des constituants isolés dans l'étude considérée. Cette efficacité peut être quantifiée, par exemple par le $\log_{10}$ des cellules tuées qui est déterminé selon la formule suivante :

$$\log_{10} \text{ des cellules tuées} = \text{T-C (jours)}/3.32 \cdot T_d$$

dans laquelle T - C représente le délai de croissance des cellules qui est le temps moyen, en jours, pour que les tumeurs du groupe traité (T) et les tumeurs du groupe témoin (C) aient atteint une valeur prédéterminée (1 g par exemple) et $T_d$ représente le temps, en jours, nécessaire au doublement du volume de la tumeur chez les animaux témoins.[T.H. CORBETT et coll., Cancer, 40, 2660.2680 (1977) ; F.M. SCHABEL et coll., Cancer Drug Development, Part B, Methods in Cancer Research, 17, 3-51, New-York, Academic Press Inc. (1979)] Un produit est considéré comme actif si $\log_{10}$ des cellules tuées est supérieur ou égal à 0,7. Un produit est considéré comme très actif si $\log_{10}$ des cellules tuées est supérieur à 2,8.

La combinaison, utilisée à sa dose maximale tolérée propre, dans laquelle chacun des constituants sera présent à une dose généralement inférieure ou égale à sa dose maximale tolérée, manifestera une synergie thérapeutique lorsque le $\log_{10}$ des cellules tuées sera supérieur à la valeur du $\log_{10}$ des cellules tuées du meilleur constituant lorsqu'il est administré seul.

L'efficacité des combinaisons sur les tumeurs solides peut être déterminée expérimentalement de la manière suivante :

Les animaux soumis à l'expérience, généralement des souris, sont greffés bilatéralement par voie sous-cutanée avec 30 à 60 mg d'un fragment de tumeur au jour 0. Les animaux portant les tumeurs sont mélangés avant d'être soumis aux divers traitements et contrôles. Dans le cas de traitement de tumeurs avancées, on laisse les tumeurs se développer jusqu'à la taille désirée, les animaux ayant des tumeurs insuffisamment développées étant éliminés. Les animaux sélectionnés sont répartis au hasard pour subir les traitements et les contrôles. Des animaux non porteurs de tumeurs peuvent être également soumis aux mêmes traitements que les animaux porteurs afin de pouvoir dissocier l'effet toxique de l'effet propre sur la tumeur. La chimiothérapie commence généralement de 3 à 22 jours après le greffage selon le type de tumeur et les animaux sont observés tous les jours. Les différents groupes d'animaux sont pesés trois ou quatre fois par semaine jusqu'à ce que la perte maximale de poids soit atteinte puis les groupes sont pesés au moins une fois par semaine jusqu'à la fin de l'essai.

Les tumeurs sont mesurées deux ou trois fois par semaine jusqu'à ce que la tumeur atteigne environ 2 g ou jusqu'à la mort de l'animal si celle-ci survient avant que la tumeur atteigne 2 g. Les animaux sont autopsiés lors du sacrifice.

L'activité antitumorale est déterminée en fonction des différents paramètres enregistrés.

Pour l'étude des combinaisons sur des leucémies, les animaux sont greffés avec un nombre déterminé de cellules et l'activité antitumorale est déterminée par l'augmentation du temps de survie des souris traitées par rapport aux témoins. Un produit est considéré comme actif si le temps d'augmentation de survie est supérieur à 27 % et il est considéré comme très actif s'il est supérieur à 75 % dans le cas de la leucémie P388.

A titre d'exemples, sont donnés, dans les tableaux suivants les résultats obtenus avec des combinaisons du Taxotère et de divers agents chimiothérapeutiques tels que le cyclophosphamide (agent alkylant), le 5-fluorouracil (antimétabolite), utilisées à leur dose optimale.

TABLEAU 1

| Activité de la combinaison Taxotère + cyclophosphamide à la dose optimale sur l'adénocarcinome mammaire MA13/C avancé greffé par voie sous-cutanée | | | | |
|---|---|---|---|---|
| Produit | Dose mg/kg/ injection i. v. | Administration aux jours : | Dose totale mg/kg | $\log_{10}$ cell. tuées |
| Taxotère | 15 | 14, 17, 20 | 45 | 2,8 |
| cyclophosphamide | 118 | 14 | 118 | 1,3 |
| Taxotère + cyclophosphamide | 7,5 90,0 | 14, 17, 20 14 | 22,5 90 | 3,4 |

TABLEAU 2

| Activité de la combinaison Taxotère + 5-fluorouracil à la dose optimale sur l'adénocarcinome colique C38 avancé greffé par voie sous-cutanée | | | | |
|---|---|---|---|---|
| Produit | Dose mg/kg/ injection i. v. | Administration aux jours : | Dose totale mg/kg | $\log_{10}$ cell. tuées |
| Taxotère | 22 | 21, 25, 29, 33 | 88,0 | 1,4 |
| 5-fluorouracil | 43,4 | 21, 25, 29, 33 | 173,6 | 1,1 |
| Taxotère + 5-fluorouracil | 17,6 27,0 | 21, 25, 29, 33 (simultanée) | 70,4 108,0 | 4,8 |

La présente invention concerne également les compositions pharmaceutiques contenant les combinaisons selon l'invention.

Les produits qui constituent la combinaison peuvent être administrés simultanément, séparément ou d'une manière étalée dans le temps de façon à obtenir le maximum d'efficacité de la combinaison ; chaque administration pouvant avoir une durée variable allant d'une administration totale rapide à une perfusion continue.

Il en résulte que, au sens de la présente invention, les combinaisons ne sont pas uniquement limitées à celles qui sont obtenues par association physique des constituants, mais aussi à celles qui permettent une administration séparée qui peut être simultanée ou étalée dans le temps.

Les compositions selon l'invention sont de préférence les compositions administrables par voie parentérale. Cependant, ces compositions peuvent être administrées par voie orale ou par voie intrapéritonéale dans le cas des thérapies loco-régionales.

Les compositions pour administration parentérale sont généralement des solutions ou des suspensions stériles pharmaceutiquement acceptables qui peuvent éventuellement être préparées extemporanément au moment de l'emploi. Pour la préparation de solutions ou de suspensions non aqueuses peuvent être utilisées des huiles végétales naturelles telles que l'huile d'olive, l'huile de sésame ou l'huile de paraffine ou les esters organiques injectables tels que l'oléate d'éthyle. Les solutions stériles aqueuses peuvent être constituées d'une solution du produit dans l'eau. Les solutions aqueuses conviennent pour l'administration intraveineuse dans la mesure où le pH est convenablement ajusté et où l'isotonicité est réalisée, par exemple par une quantité suffisante de chlorure de sodium ou de glucose. La stérilisation peut être réalisée par chauffage ou par tout autre moyen qui n'altère pas la composition. Les combinaisons peuvent aussi se présenter sous forme de liposomes ou sous forme d'association avec des supports tels que les cyclodextrines ou les polyéthylèneglycols.

Les compositions pour administration orale ou intrapéritonéale sont, de préférence, des solutions ou des suspensions aqueuses.

Dans les combinaisons selon l'invention dont l'application des constituants peut être simultanée, séparée ou étalée dans le temps, il est particulièrement avantageux que la quantité de dérivé du taxane représente de 10 à 90 % en poids de la combinaison cette teneur pouvant varier en fonction de la nature de la substance associée, de l'efficacité recherchée et de la nature du cancer à traiter.

Les combinaisons selon l'invention sont particulièrement utiles dans le traitement des cancers du sein, de l'ovaire ou du poumon. En particulier, elles peuvent présenter l'avantage de pouvoir mettre en oeuvre les constituants à des doses nettement plus faibles que celles auxquelles ils sont utlilisés seuls.

**Revendications**

1. Combinaisons du Taxotère et de ses analogues avec les complexes de coordination du platine utiles dans le traitement des maladies néoplastiques.

2. Combinaisons selon la revendication 1 **caractérisées en ce que** les complexes de coordination du platine sont choisis parmi le carboplatine et le cisplatine.

3. Combinaisons selon l'une des revendications 1 et 2 **caractérisées en ce qu'**elles contiennent en outre des facteurs de croissance de type hématopoïétiques.

4. Combinaisons selon l'une des revendications 1 à 3 **caractérisées en ce qu'**elles contiennent de 10 à 90 % en poids de Taxotère.

5. Produits contenant du Taxotère et au moins une substance thérapeutiquement utile telle que définie dans l'une des revendications 1 à 3 dans le traitement des maladies néoplastiques comme préparation combinée pour une utilisation simultanée, séparée ou étalée dans le temps en thérapie anticancéreuse.

6. Combinaisons du Taxol et de ses analogues avec les complexes de coordination du platine.

7. Combinaisons selon la revendication 6 **caractérisées en ce que** les complexes de coordination du platine sont choisis parmi le carboplatine et le cisplatine.

8. Combinaisons selon l'une des revendications 6 et 7 **caractérisées en ce qu'**elles contiennent en outre des facteurs de croissance de type hématopoïétiques.

9. Combinaisons selon l'une des revendications 6 et 7 **caractérisées en ce qu'**elles contiennent de 10 à 90 % en poids de Taxol.

10. Produits contenant du Taxol et au moins une substance thérapeutiquement utile telle que définie dans l'une des revendications 6 et 7 dans le traitement des maladies néoplastiques comme préparation combinée pour une utilisation simultanée, séparée ou étalée dans le temps en thérapie anticancéreuse.

11. Procédé de préparation d'une combinaison selon l'une quelconque des revendications précédentes pour le traitement des cancers du sein, de l'ovaire ou du poumon.

| | Office européen des brevets | **RAPPORT DE RECHERCHE EUROPEENNE** | Numéro de la demande EP 02 02 8931 |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X,D | DATABASE WPI<br>Section Ch, Week 199210<br>Derwent Publications Ltd., London, GB;<br>Class B04, AN 1992-079710<br>XP002161505<br>* abrégé *<br>& US 7 696 923 A (US DEPT OF HEALTH)<br>21 janvier 1992 (1992-01-21) | 1,2,5-7,<br>10,11 | A61K31/337 |
| X,P,<br>D | WO 92 19765 A (US DEPT. OF HEALTH)<br>12 novembre 1992 (1992-11-12)<br>* revendications 19-21 *<br>* figures 4,8,9 *<br>* tableaux 3,4 *<br>* exemples 3,5 * | 1,2,5-7,<br>10,11 | |
| E | EP 0 577 215 A (STERLING WINTHROP)<br>5 janvier 1994 (1994-01-05)<br>* revendications 1,4,15,19-21,29-32 *<br>* page 3, ligne 9 *<br>* page 3, colonne 21 *<br>* page 3, colonne 29 *<br>* exemples 11-16 * | 1,2,5-7,<br>10,11 | |

DOMAINES TECHNIQUES RECHERCHES (Int.Cl.7)

A61K

Le présent rapport a été établi pour toutes les revendications

| Lieu de la recherche | Date d'achèvement de la recherche | Examinateur |
|---|---|---|
| LA HAYE | 29 janvier 2003 | Scarponi, U |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**  EP 02 02 8931

La présente annexe indique les membres de la famille de brevets relatifs aux documents brevets cités dans le rapport de
recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

29-01-2003

| Document brevet cité<br>au rapport de recherche | | Date de<br>publication | | Membre(s) de la<br>famille de brevet(s) | | Date de<br>publication |
|---|---|---|---|---|---|---|
| WO 9219765 | A | 12-11-1992 | AU | 2005692 | A | 21-12-1992 |
| | | | WO | 9219765 | A1 | 12-11-1992 |
| | | | US | 5645988 | A | 08-07-1997 |
| | | | US | 6150398 | A | 21-11-2000 |
| EP 577215 | A | 05-01-1994 | US | 5399363 | A | 21-03-1995 |
| | | | AT | 190835 | T | 15-04-2000 |
| | | | AU | 675432 | B2 | 06-02-1997 |
| | | | AU | 4156093 | A | 06-01-1994 |
| | | | CA | 2098242 | A1 | 02-01-1994 |
| | | | CN | 1084391 | A ,B | 30-03-1994 |
| | | | CZ | 9301316 | A3 | 16-02-1994 |
| | | | DE | 69328136 | D1 | 27-04-2000 |
| | | | DE | 69328136 | T2 | 09-11-2000 |
| | | | DK | 577215 | T3 | 28-08-2000 |
| | | | EP | 0577215 | A1 | 05-01-1994 |
| | | | ES | 2143488 | T3 | 16-05-2000 |
| | | | FI | 933040 | A | 02-01-1994 |
| | | | HU | 64832 | A2 | 28-03-1994 |
| | | | JP | 7165562 | A | 27-06-1995 |
| | | | MX | 9303950 | A1 | 31-01-1994 |
| | | | NO | 932403 | A | 03-01-1994 |
| | | | NZ | 248042 | A | 26-10-1994 |
| | | | RU | 2130781 | C1 | 27-05-1999 |
| | | | SG | 55089 | A1 | 21-12-1998 |
| | | | SK | 68193 | A3 | 02-02-1994 |
| | | | US | 5494683 | A | 27-02-1996 |

EPO FORM P0460

Pour tout renseignement concernant cette annexe : voir Journal Officiel de l'Office européen des brevets, No.12/82